# EUROPEAN PATENT SPECIFICATION

(11) **EP 2 711 028 B1**
(45) Date of publication and mention of the grant of the patent: **20.03.2019**
(21) Application number: 12782257.5
(22) Date of filing: 20.03.2012
(51) Int. Cl.: A61L 15/28, C08J 5/18, D06M 15/13

(54) **FILM CONTAINING ALGINATE MEMBRANE LAYER AND METHOD FOR PREPARING SAME**
FILM MIT EINER ALGINATMEMBRANSCHICHT UND VERFAHREN ZU SEINER HERSTELLUNG
FILM CONTENANT UNE MEMBRANE D'ALGINATE ET PROCÉDÉ DE PRÉPARATION ASSOCIÉ

(30) Priority: 11.05.2011 CN 201110120855
(43) Date of publication of application: 26.03.2014
(73) Proprietor: Lin, Yu-Yueh, New Taipei City (TW)
(72) Inventor: CHIU, Yao-Chung, New Taipei City (TW)
(74) Representative: Lang, Christian
(86) International application number: PCT/CN2012/000350
(87) International publication number: WO 2012/152054

(56) References cited:
- EP-A1- 2 111 926
- EP-A2- 0 243 069
- CN-A- 1 915 440
- CN-A- 101 033 564
- JP-A- H02 311 535
- JP-A- H02 311 536
- US-A- 5 470 576
- US-B1- 6 372 248

## Description

### BACKGROUND OF THE INVENTION

The present invention relates to a film containing at least one alginate membrane and a manufacturing method of the same. The film includes a film carrier and an alginate membrane. The film carrier absorbs liquid. At least a layer of alginate solution and a layer of salt water solution containing divalent metal ions are coated on a surface of the film carrier by two separate and continuous processes. Then crosslinking occurs between the two solutions on the surface of the film carrier to produce the film containing an alginate membrane.

Alginic acid is a natural copolymer, easily reacted with metal ions from salts to form various kinds of alginate for commercial uses. The water-soluble sodium alginate reacts with double charged calcium ion (Ca²⁺) from salts so as to form insoluble calcium alginate gel. Alginate products for commercial use include sodium alginate, potassium alginate, ammonium alginate, etc, being applied to wound dressings or facial masks. There are several methods for making alginate gels available now, including:
(1) dialysis /diffusion method: this is the most commonly used method. The alginate solution is gelled by diffusion of calcium ions from an outer reservoir.
(2) in situ gelling: a calcium salt with limited solubility is mixed with a sodium alginate solution and then a slowly acting acid is added into the mixture. Due to the acid, the calcium ions are released to cross-link the alginate molecules so as to form calcium alginate gel.
(3) cooling method: at high temperature, calcium ions are unable to bond with alginic acid. Thus, dissolve sodium alginate and calcium salts in hot solution. Then allow the solution to set and form gel by cooling.
(4) crosslinking method: the hydrogen groups contained in alginate molecules are cross-linked by epichlorohydrin (ECH) so as to form gel insoluble in solution.

Now the alginate or similar materials have been applied to wound dressings or facial masks, as the alginate revealed in US 6,080,420, US 6,258,995, US 6,203,845, US 6,201,164, US 6,372,248, US 6,326,524, US 5,144,016, US 5,230,853, US 5,622,666, US 5,660,857, US 5,675,957, GB 9419572, GB 9501514, GB 9516930, PCT/ GB 9502284 (WO96/10106), PCT/ GB 9601719 (WO97/03710), PCT/ GB 9701098 (WO97/39781), PCT/ DK 9700292 (WO98/02196), WO2008/072817, TW 95218502, TW201100119A1, TW 1265814, etc. However, most of these prior arts focus on components of the materials or percent by weight of the components. JP H02 311 536 A describes a method for producing a hydrous alginic acid film reinforced with a nonwoven fabric by casting an alginate aqueous solution on a nonwoven fabric and spraying calcium salt aqueous solution thereon. JP H02 311 535 A relates to a method for producing a water-containing alginic acid film by casting a soluble alginate aqueous solution on an aqueous soluble calcium salt solution supported on a base material. The problems occur or practical requirements during manufacturing of the alginate membrane have not been mentioned, especially how to mass produce the alginate membrane or how to reduce manufacturing cost of the alginate membrane.

Thus, there is a need to provide a film containing an alginate membrane and a manufacturing method of the same related to water-soluble sodium alginate or potassium alginate for improving the performance and applications of the film. The manufacturers in this industry have more options.

### SUMMARY OF THE INVENTION

Therefore, it is a primary object of the present invention to provide a film containing at least one alginate membrane and a manufacturing method of the same. The film includes a film carrier and at least one alginate membrane. The film carrier can be non-woven fabric (non-woven cloth), textile, a porous sponge film, or a plastic film. The film carrier is made from synthetic fibers, natural fibers, one kind of high molecular polymers or their combinations. The synthetic fibers include polyethylene terephthalate (PET), nylon, acrylics, polypropylene (PP), poly lactic acid (PLA), etc. The natural fibers include cotton, linen, wool, silk, etc. The alginate membrane is a hydrogel complex membrane having a network structure, formed by following steps: provide an alginate solution containing a certain weight of sodium alginate or potassium alginate (in weight percent, wt%) and a salt water solution containing a certain weight of divalent metal ions such as calcium ions. The salt can be calcium lactate, calcium chloride, calcium gluconate, poly-glutamic acid calcium, calcium carbonate, calcium sulfate, etc. Then at least one surface of the film carrier is coated with a layer of the alginate solution and a layer of the salt water solution by at least two separate and continuous processes. There is no restriction on the order of the coating solution, provided that the first process is carried out by soaking and the second process is carried out by spray - coating. Then crosslinking occurs between alginate and the divalent metal ions in the salt water solution, on the surface of the film carrier or infiltrating into the film carrier to form a hydrogel complex membrane having a network structure. Thus, a film containing an alginate membrane is mass-produced continuously and quickly. Therefore, the production cost is reduced due to mass-production of the alginate membrane.

It is another object of the present invention to provide a film containing at least one alginate membrane and a manufacturing method of the same. When the surface of the film carrier (such as non-woven fabric (cloth), textile porous sponge film, etc.) is a rough surface, the alginate membrane formed is connected to the film carrier tightly, not separated easily. When the surface of the film carrier (such as plastic film) is a smooth surface, the alginate membrane formed is able to be separated from the film carrier and used for following processes.

It is a further object of the present invention to provide a film containing at least one alginate membrane and a manufacturing method of the same. After the crosslinking reaction being completed, one or two surfaces of the film carrier is/are spray-coated continuously with a layer of alginate solution and a layer of salt water solution in sequence. Thus the thickness of the alginate membrane formed is easy to be increased and controlled by at least one further crosslinking reaction.

### BRIEF DESCRIPTION OF THE DRAWINGS

Fig. 1 is a schematic drawing showing a cross section of an embodiment having an alginate membrane disposed on a surface of a film carrier according to the present invention;
Fig. 2 is a schematic drawing showing a cross section of an embodiment having an alginate membrane disposed on a surface of a film carrier according to the present invention;
Fig. 3 is a schematic drawing showing arrangement of coating equipments during manufacturing processes of the present invention;

### DETAILED DESCRIPTION OF THE PREFFERED EMBODIMENT

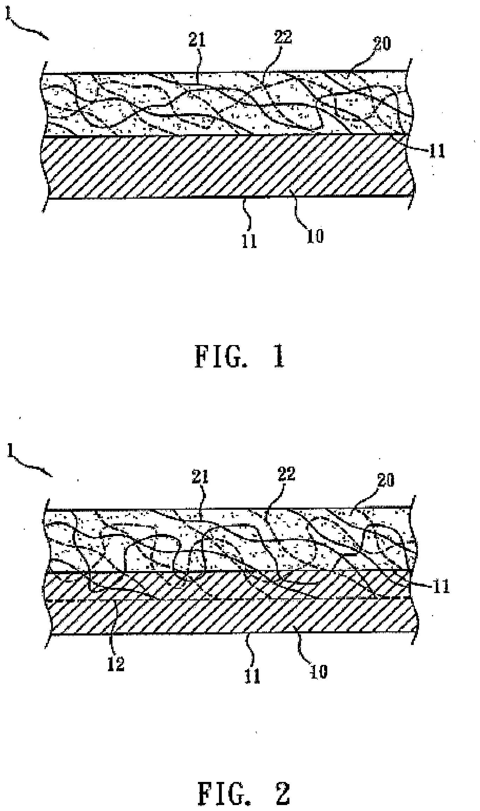

Refer to Fig. 1 and Fig. 2, a film 1 containing at least one alginate membrane according to the present invention includes a film carrier 10, and at least one alginate membrane 20.The film carrier 10 is a carrier that absorbs liquid. The film carrier 10 can be a plastic film with a smooth surface or a thin layer with a rough surface such as non-woven fabric (cloth), fabric or porous sponge. Moreover, the film carrier 10 is made from synthetic fibers, natural fibers, one kind of high molecular polymers or combinations of at least two kinds of high molecular polymers. The synthetic fibers include but not limited to polyethylene terephthalate (PET), nylon, acrylics, polypropylene (PP), poly lactic acid (PLA), etc. The natural fibers include but not limited to cotton, linen, wool, silk, etc. A surface 11 of the film carrier 10 can be a rough surface or a smooth surface according to users' needs or applications.

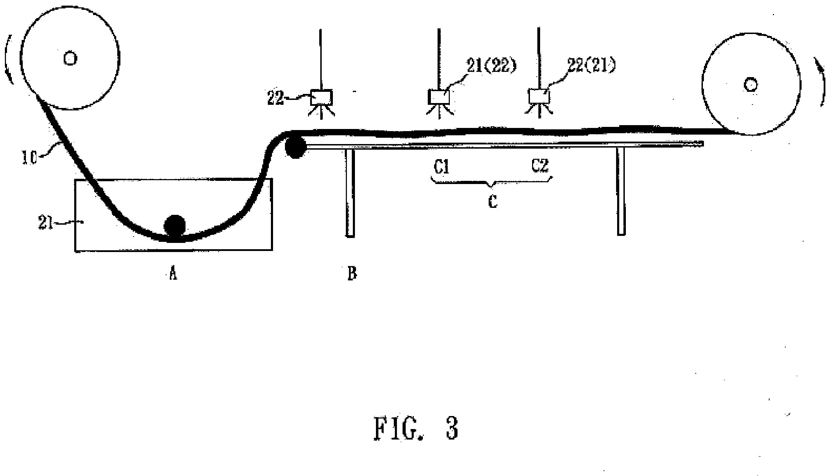

The alginate membrane 20 is a hydrogel complex membrane having a network structure. Refer to Fig. 3, the alginate membrane 20 is produced by following steps:
providing an alginate solution 21 containing a certain weight percent (wt%) of sodium alginate or potassium alginate and a salt water solution 22 containing a certain weight percent (wt%) of divalent metal ions such as calcium ions;
coating a layer of the alginate solution 21 and a layer of the salt water solution 22 evenly on at least one surface 11 of a film carrier 10 by two separate processes carried out successively with the first process being carried out by soaking and the second process being carried out by spray - coating;
crosslink alginate in the alginate solution 21 with divalent metal ions in the salt water solution 22 on the surface 11 of the film carrier 10 so as to form a hydrogel complex membrane (20) with a network structure on the surface 11 of the film carrier 10 (as shown in Fig. 1), or filtrating into the surface 11 of the film carrier 10 (as shown in Fig. 2). As shown in Fig. 1 and Fig. 2, the alginate membrane 20 is formed by alginate (represented by solid lines) crosslinked with divalent metal ions (represented by dotted lines) to have a crosslinked network structure.

In the present invention, the surface 20 of the film carrier 10 is used to load the alginate solution 21 and the salt water solution 22 so that an alginate membrane 20 such as a calcium alginate membrane is mass-produced on the surface 11 of the film carrier 10 fast and continuously. Therefore, the manufacturing cost is reduced due to mass-production of the alginate membrane.

Refer to Fig. 3, a manufacturing method of the film 1 containing at least one alginate membrane of the present invention includes following steps:
(a) providing a film carrier 10 that absorbs liquid, an alginate solution 21 containing a certain percent of sodium alginate or potassium alginate by weight (wt%) and a salt water solution 22 containing a certain percent of divalent metal ions such as calcium ions by weight(wt%);
(b) using at least two separate and successive processes to coat a layer of the alginate solution 21 and a layer of the salt water solution 22 respectively on at least one surface 11 of the film carrier 10;
(c) crosslinking the alginate in the alginate solution 21 with the divalent metal ions in the salt water solution 22 on the surface 11 of the film carrier 10 so as to form a hydrogel complex membrane 20 having a network structure, formed on the surface 11 of the film carrier 10, as show in Fig. 1 or filtrating into the surface 11, as shown in Fig. 2.

In the above step (b), the order of coating the alginate solution 21 or the salt water solution 22 on the surface 11 of the film carrier 10 on at least one surface 11 of the film carrier 10 is not limited.

In the step (b), firstly at least one surface 11 of the film carrier 10 is coated with a layer of the alginate solution 21. Then a layer of the salt water solution 22 is coated over the layer of the alginate solution 21. While crosslinking the alginate in the alginate solution 21 with the divalent metal ions in the salt water solution 22 on the surface 11 of the film carrier 10, the divalent metal ions in the salt water solution 22 generally infiltrate further into an inner surface of the surface 11 for crosslinking of the alginate. Thus a hydrogel complex membrane 20 with a network structure shown in Fig. 2 is formed above or in the surface 11 of the film carrier 10. In the Fig. 2, the alginate membrane 20 is infiltrated into an infiltration interface 12. The position of the infiltration interface 12 in the surface layer 11 of the film carrier 10 is not limited, depending on the relative concentration of the alginate solution 21 and the salt water solution 22. In this embodiment, the alginate membrane 20 is not separated from the film carrier 10 easily.
According to another embodiment, in the step (b), at least one surface 11 of the film carrier 10 is coated with a layer of the salt water solution 22 and then a layer of an alginate solution 21 is coated over the layer of the salt water solution 22. While crosslinking the alginate in the alginate solution 21 with the divalent metal ions in the salt water solution 22 on the surface 11 of the film carrier 10, the alginate in the alginate solution 21 is firstly crosslinked over the surface 11 of the film carrier 10 to form a hydrogel complex membrane (20) with larger density. Thus the infiltration interface 12 is overlapped with the surface 11, not infiltrating into the inner surface of the surface 11, to crosslink with the divalent metal ions. The hydrogel complex membrane (20) having a network structure is only formed on the upper surface 11 of the film carrier 10, as shown in Fig. 1. The hydrogel complex membrane (20) is the alginate membrane 20 formed on the upper surface 11 of the film carrier 10 in Fig. 1. In this embodiment, the alginate membrane 20 is easy to be separated from the film carrier 10.

Moreover, for convenience of arrangement of coating equipments and coating processes, in the step (b), the first coating is performed by soaking the film carrier 10 into the solution while the second coating is run by spray coating. Take the equipment arrangement and coating procedures shown in Fig. 3 as an example. A roller of film carrier 10 absorbing liquid and having certain length is transported and soaked into a tank filled with an alginate solution 21 by a conveyor. This is the soaking process A shown in the Fig. 3. In this embodiment, the alginate solution 21 is produced by a certain amount of sodium alginate dissolved in water. The weight percent of the sodium alginate in solution ranges from 0.01 to 20 wt% while 1-5 wt% is preferred. In this embodiment, an upper and a lower surfaces (11) of the film carrier 10 are evenly coated with a layer of sodium alginate solution (21). Then the conveyor sends the film carrier 10 already soaked with the sodium alginate solution to be coated with a layer of salt water solution 22 containing a certain weight percent of divalent metal ions by a spray coating process B. In this embodiment, the salt water solution 22 contains divalent calcium ions. The weight percent of the divalent metal ions in the salt water solution ranges from 0.01 to 50 wt% while 1-10 wt% is preferred. The salt water solution 22 is coated over the layer of sodium alginate solution on the surface 11 of the film carrier 10 by spray coating. The salt water solution 22 containing divalent metal ions may further infiltrate into the film carrier 10 and react with the sodium alginate solution in the film carrier 10 for crosslinking, as shown in Fig. 2.

Furthermore, according to the requirement of the thickness of the alginate membrane 20, a plurality sets of spray coating process C is designed. Each set of spray coating process C includes at least one spray coating of the alginate solution 21 C1 and at least one spray coating of the slat water solution C2. After the completion of the spray coating process B, perform the spray coating processes of the alginate solution 21 and the salt water solution in sequence on one surface or two surfaces of the film carrier 10 until the alginate membrane 20 is having the thickness required.

The structure and formation of the film 1 containing at least one alginate membrane 20 of the present invention are revealed in details by following embodiments.

### < Embodiment 1 (reference example) >

A film carrier 10 is non-woven fabric or cloth made from polypropylene (PP) and is used to absorb 4 wt% calcium lactate that is used as the salt water solution 22. Then 2 wt% sodium alginate solution used as the alginate solution 21 and having the thickness of 1.1mm is coated over the film carrier 10 with the calcium lactate. After crosslinking, a layer of the alginate membrane 20 with the thickness of about 1mm is formed on the PP non-woven fabric. The alginate membrane 20 is a hydrogel complex membrane having a network structure. Moreover, when the PP non-woven fabric contacts the salt water solution 22 and the alginate solution 21 by a rough surface of the PP non-woven fabric, the alginate membrane 20 formed is connected with the non-woven fabric (the film carrier 10). When the PP non-woven fabric contacts the salt water solution 22 and the alginate solution 21 by a smooth surface of the PP non-woven fabric, the alginate membrane 20 formed is separated from the non-woven fabric (the film carrier 10).

### < Embodiment 2 >

A film carrier 10 is non-woven fabric or cloth made from polypropylene (PP) and nylon and having the thickness of 0.42mm. The film carrier 10 absorbing liquid is soaked into 2wt% sodium alginate solution continuously. After removing extra solution, coat 4 wt% calcium lactate solution on an upper and lower surfaces (11) of the non-woven fabric (the film carrier 10) by spray coating so as to produce a film 1 containing an alginate membrane 20 and having the thickness of about 0.72mm continuously. This thickness of the film 1 includes the thickness of the fabric. If user wants to produce thicker alginate membrane 20, after the calcium lactate reacting with the sodium alginate, continuously coat one surface or two surfaces of the film 1 produced with a layer of sodium alginate solution and a layer of calcium lactate solution for crosslinking of alginate. Repeat the above procedures until the thickness of the film 1 achieves the required thickness.

### < Embodiment 3 >

A film carrier 10 is non-woven fabric or cloth made from polypropylene (PP) and nylon and having the thickness of 0.42mm. The film carrier 10 absorbing liquid is soaked into 4wt% calcium lactate solution continuously. After removing extra solution, coat 2 wt% sodium alginate solution on an upper and lower surfaces (11) of the non-woven fabric (the film carrier 10) by spray coating so as to produce a film 1 containing an alginate membrane 20 and having the thickness of about 0.72mm continuously. This thickness of the film 1 includes the thickness of the fabric. If user wants to produce thicker alginate membrane 20, after the sodium alginate reacting with the calcium lactate, continuously coat one surface or two surfaces of the film 1 with a layer of calcium lactate solution and a layer of sodium alginate solution for crosslinking of alginate. Repeat the above procedures until the thickness of the film 1 achieves the required thickness.

In addition, while producing the film containing alginate membrane of the present invention, the alginate solution 21 can be added with other materials (additives) such as bioactive components in cosmetics or pigments, vitamins, growth factors, peptides, proteins, minerals, etc. The bioactive components include nutrients in cosmetics, essences in cosmetics, and drug solutions in cosmetics. The pigments are animal/plant extracts.

Compared with the technique available now, a film containing at least one alginate membrane and a manufacturing method of the same have following advantages:
(1) The film can be produced continuously and quickly. This beneficial to mass-production of the alginate membrane and the manufacturing cost is reduced.
(2) The film can be produced by general coating equipments so that the cost of production equipments can be reduced.
(3) After the first crosslinking reaction being completed, at least one surface of the film carrier is spray-coated continuously with a layer of alginate solution and a layer of salt water solution in sequence. Thus a further crosslinking reaction occurs on the surface of the film carrier. The thickness of the alginate membrane is easy to be increased and controlled by at least one further crosslinking reaction. Therefore the performance and applications of the alginate membrane are improved.

## Claims

1. A film (1) comprising a film carrier (10) and at least one alginate membrane (20); wherein the film carrier (10) is a carrier that absorbs liquid;
the alginate membrane (20) is a hydrogel membrane having a network structure formed by crosslinking occurring between alginate in at least one layer of alginate solution (21) and the divalent metal ions of at least one layer of salt water solution (22) coated on at least one surface (11) of the film carrier (10); the alginate solution (21) containing a certain percent of sodium alginate or potassium alginate by weight while the salt water solution (22) containing a certain percent of divalent metal ions by weight; the layer of the alginate solution (21) and the layer of the salt water solution (22) being coated on the surface (11) of the film carrier (10) by two separate and continuous coating processes, wherein the alginate membrane (20) is formed on and / or penetrating into the surface of the film carrier (10),
wherein the alginate membrane (20) is not easily separable from the film carrier (10) after firstly coating at least one surface (11) of the film carrier (10) with a layer of the alginate solution (21) by soaking and subsequent coating with a layer of the salt water solution (22) over the layer of the alginate solution (21) by spray-coating, wherein the divalent metal ions in the salt water solution (22) being infiltrated into an inner surface of the film carrier (10), so that the alginate hydrogel membrane (20) having a network structure is formed above the surface (11) of the film carrier (10) and in an infiltration interface (12) penetrating into a surface region of the film carrier (10), such that the alginate membrane (20) being infiltrated into the film carrier (10), wherein the position of the infiltration interface (12) is dependent on the relative concentration of the alginate solution (21) and the salt water solution (22);
or wherein the hydrogel membrane (20) is separable from the film carrier (10) after firstly coating at least one surface (11) of the film carrier (10) with a layer of the salt water solution (22) by soaking and a second coating with a layer of an alginate solution (21) over the layer of the salt water solution (22) by spray-coating, wherein the alginate in the alginate solution (21) being crosslinked with the divalent metal ions in the salt water solution (22) over the surface (11) of the film carrier (10) to form a hydrogel membrane (20) with larger density, wherein the infiltration interface (12) being overlapped with the surface (11) and not infiltrating into the inner surface of the film carrier (10), wherein the hydrogel membrane (20) having a network structure only formed on the upper surface (11) of the film carrier (10).

2. The film as claimed in claim 1, wherein the film carrier (10) is non-woven fabric, textile, a porous sponge film, or a plastic film.

3. The film as claimed in claim 1, wherein the film carrier (10) is made from synthetic fibers, natural fibers, high molecular polymers or their combinations.

4. The film as claimed in claim 3, wherein the synthetic fibers include polyethylene terephthalate (PET), nylon, acrylics, polypropylene (PP), and poly lactic acid (PLA).

5. The film as claimed in claim 1, wherein the certain percent of sodium alginate or potassium alginate in the alginate solution (21) ranges from 0.01% to 20% by weight.

6. The film as claimed in claim 1, wherein the certain percent of sodium alginate or potassium alginate in the alginate solution (21) ranges from 1% to 5% by weight.

7. The film as claimed in claim 1, wherein the salt water solution (22) containing a certain percent of the divalent metal ions by weight includes calcium lactate solution, calcium chloride solution, calcium gluconate solution, poly-glutamic acid calcium solution, calcium carbonate solution, and calcium sulfate solution.

8. The film as claimed in claim 1, wherein the certain percent of the divalent metal ions ranges from 0.01% to 50% by weight.

9. The film as claimed in claim 1, wherein the certain percent of the divalent metal ions ranges from 1% to 10% by weight.

10. The film as claimed in claim 1, wherein the alginate solution (21) is added with additives selected from the group consisting of including nutrients for use in cosmetics, essences for use in cosmetics, and drug solutions for use in cosmetics or pigments.

11. A manufacturing method of a film (1) comprising a film carrier (10) and at least one alginate membrane (20) comprising the steps of:
(a) providing a film carrier (10) that absorbs liquid, an alginate solution (21) containing a certain percent of sodium alginate or potassium alginate by weight and a salt water solution (22) containing a certain percent of divalent metal ions by weight;
(b) using at least two separate and successive processes to coat a layer of the alginate solution (21) and a layer of the salt water solution (22) respectively on at least one surface (11) of the film carrier (10), wherein the first coating process is performed by soaking; and wherein the second coating process is performed by spray coating;
(c) crosslinking the alginate in the alginate solution (21) with the divalent metal ions in the salt water solution (22) on the surface (11) of the film carrier (10) so as to produce an alginate membrane (20) that is a hydrogel membrane having a network structure, formed on the surface (11) of the film carrier (10), or formed infiltrating into the surface (11) of the film carrier (10);
wherein in the step (b), firstly at least one surface (11) of the film carrier (10) is coated with a layer of the alginate solution (21) and then a layer of the salt water solution (22) is coated over the layer of the alginate solution (21), wherein the divalent metal ions in the salt water solution (22) will infiltrate into an inner surface of the film carrier (10) for crosslinking of the alginate within an infiltration interface (12), wherein a hydrogel membrane (20) with a network structure is formed above or in the surface (11) of the film carrier (10), so that the alginate membrane (20) is infiltrated into the infiltration interface (12) and being not easily separable from the film carrier (10), wherein the position of the infiltration interface (12) depends on the relative concentration of the alginate solution (21) and the salt water solution (22);
or wherein in the step (b), at least one surface (11) of the film carrier (10) is first coated with a layer of the salt water solution (22) and then a layer of an alginate solution (21) is coated over the layer of the salt water solution (22), wherein the alginate in the alginate solution (21) is firstly crosslinked at the surface (11) of the film carrier (10) to form a hydrogel membrane (20) with larger density, so that the alginate membrane (20) is overlapped with the surface (11) and is not infiltrating into the inner surface of the film carrier (10), so that the hydrogel complex alginate membrane (20) having a network structure is only formed on the upper surface (11) of the film carrier (10), so that the alginate membrane (20) is separable from the film carrier (10).

12. The method as claimed in claim 11, wherein the film carrier (10) is non-woven fabric, textile, a porous sponge film, or a plastic film.

13. The method as claimed in claim 11, wherein the film carrier (10) is made from synthetic fibers, natural fibers, high molecular polymers or their combinations.

14. The method as claimed in claim 11, wherein the synthetic fibers include polyethylene terephthalate (PET), nylon, acrylics, polypropylene (PP), and poly lactic acid (PLA).

15. The method as claimed in claim 11, wherein the certain percent of sodium alginate or potassium alginate in the alginate solution (21) ranges from 0.01% to 20% by weight.

16. The method as claimed in claim 11, wherein the certain percent of sodium alginate or potassium alginate in the alginate solution (21) ranges from 1% to 5% by weight.

17. The method as claimed in claim 11, wherein the salt water solution (22) containing a certain percent of the divalent metal ions by weight includes calcium lactate solution, calcium chloride solution, calcium gluconate solution, poly-glutamic acid calcium solution, calcium carbonate solution, and calcium sulfate solution.

18. The method as claimed in claim 11, wherein the certain percent of the divalent metal ions ranges from 0.01% to 50% by weight.

19. The method as claimed in claim 11, wherein the certain percent of the divalent metal ions ranges from 1% to 10% by weight.

20. The method as claimed in claim 11, wherein in the step (b), the second coating is performed by spray coating.

21. The method as claimed in claim 11, wherein after the crosslinking in the step (c) being completed, the method further includes a step of performing a spray coating process of the alginate solution (21) and a spray coating process of the salt water solution (22) in order on the surface (11) of the film carrier (10) for crosslinking and increasing thickness of the alginate membrane (20).

22. The method as claimed in claim 11, wherein an alginate solution (21) is added with additives selected from the group consisting of nutrients for use in cosmetics, essences for use in cosmetics, and drug solutions for use in cosmetics or pigments.

## Patentansprüche

1. Film (1), der einen Filmträger (10) und zumindest eine Alginatmembran (20) umfasst, wobei der Filmträger (10) ein Träger ist, der Flüssigkeit absorbiert;
die Aginatmembranschicht (20) ist eine Hydrogelmembran, die eine Netzwerkstruktur aufweist, die durch Querverbindungen ausgebildet wird, die zwischen Alginat in zumindest einer Schicht der Alginatlösung (21) und den divalenten Metallionen zumindest einer Schicht der Salzwasserlösung (22), die an die zumindest eine Oberfläche (11) des Filmträgers (10) aufgeschichtet ist, auftreten; die Alginatlösung (21) enthält auf das Gewicht bezogen einen gewissen Prozentsatz an Natriumalginat oder Kaliumalginat, während die Salzwasserlösung (22) auf das Gewicht bezogen einen gewissen Prozentsatz an divalenten Metallionen enthält; die Schicht der Alginatlösung (21) und die Schicht der Salzwasserlösung (22) werden an der Oberfläche (11) des Filmträgers (10) durch zwei separate und kontinuierliche Beschichtungsprozesse aufgeschichtet, wobei die Alginatmembran (20) an der Oberfläche des Filmträgers (10) ausgebildet wird und / oder in die Oberfläche des Filmträgers dringt,
wobei die Alginatmembran (20) nicht leicht von dem Filmträger (10) zu trennen ist, nach dem erstmaligen Beschichten zumindest einer Oberfläche (11) des Filmträgers (10) mit einer Schicht der Alginatlösung (21) durch Eintauchen und darauf folgendes Beschichten mit einer Schicht der Salzwasserlösung (22) über die Schicht der Alginatlösung (21) durch Sprühbeschichtung, wobei die divalenten Metallionen in der Salzwasserlösung (22) in eine innere Oberfläche des Filmträgers (10) infiltriert werden, so dass die Alginathydrogelmembran (20), die eine Netzwerkstruktur aufweist, über der Oberfläche (11) des Filmträgers (10) und in einer Infiltrationsschnittstelle (12), die in eine Oberflächenregion des Filmträgers (10) dringt, ausgebildet wird, so dass die Alginatmembran (20) in den Filmträger (10) infiltriert wird, wobei die Position der Infiltrationsschnittstelle (12) abhängig ist von der relativen Konzentration der Alginatlösung (21) und der Salzwasserlösung (22);
oder wobei die Hydrogelmembran (20) von dem Filmträger (10) trennbar ist, nach erstmaligem Beschichten zumindest einer Oberfläche (11) des Filmträgers (10) mit einer Schicht der Salzwasserlösung (22) durch Eintauchen und einem zweiten Beschichten mit einer Schicht der Alginatlösung (21) über die Schicht der Salzwasserlösung (22) durch Sprühbeschichtung, wobei das Alginat in der Alginatlösung (21) mit den divalenten Metallionen in der Salzwasserlösung (22) über die Oberfläöche (11) des Filmträgers (10) quervernetzt ist, um eine Hydrogelmembran (20) mit größerer Dichte auszubilden, wobei die Infiltrationsschnittstelle (12) mit der Oberfläche (11) überlappt ist und nicht in die innere Oberfläche des Filmträgers (10) infiltriert, wobei die Hydrogelmembran (20) eine Netzwerkstruktur aufweist, die nur an der oberen Oberfläche (11) des Filmträgers (10) ausgebildet ist.

2. Film nach Anspruch 1, bei dem der Filmträger (10) ein Vlies, Textil, ein poröser Schwamm oder ein Plastikfilm ist.

3. Film nach Anspruch 1, bei dem der Filmträger (10) aus synthetischen Fasern, natürlichen Fasern, hochmolekularen Polymeren oder ihren Kombinationen hergestellt ist.

4. Film nach Anspruch 3, bei dem die synthetischen Fasern Polyethylenterephthalate (PET), Nylon, Acryl, Polypropylen (PP) und Polymilchsäure (PLA) umfassen.

5. Film nach Anspruch 1, bei dem der gewisse auf das Gewicht bezogene Prozentsatz an Natriumalginat oder Kaliumalginat in der Alginatlösung (21) im Bereich von 0,01% bis 20% liegt.

6. Film nach Anspruch 1, bei dem der gewisse auf das Gewicht bezogene Prozentsatz an Natriumalginat oder Kaliumalginat in der Alginatlösung (21) im Bereich von 1 % bis 5% liegt.

7. Film nach Anspruch 1, bei dem die Salzwasserlösung (22), die auf das Gewicht bezogen einen gewissen Prozentsatz der divalenten Metallionen enthält, Kalziumlactatlösung, Kalziumchloridlösung, Kalziumgluconatlösung, Polyglutaminsäurekalziumlösung, Kalziumkarbonatlösung und Kalziumsulfatlösung enthält.

8. Film nach Anspruch 1, bei dem der gewisse auf das Gewicht bezogene Prozentsatz der divalenten Metallionen im Bereich von 0,01% bis 50% liegt

9. Film nach Anspruch 1, bei dem der gewisse auf das Gewicht bezogene Prozentsatz der divalenten Metallionen im Bereich von 1% bis 10% liegt.

10. Film nach Anspruch 1, bei dem die Alginatlösung (21) mit Additiven zugesetzt wird, die aus der Gruppe ausgewählt sind, die aus Nährstoffen zum Gebrauch in der Kosmetik, Essenzen zum Gebrauch in der Kosmetik, und Wirkstofflösungen zum Gebrauch in der Kosmetik oder Pigmenten besteht.

11. Verfahren zur Herstellung eines Films (1), der einen Filmträger (10) und zumindest eine Alginatmembran (20) umfasst, die die Schritte umfasst:
(a) Bereitstellen eines Filmträgers (10), der Flüssigkeit absorbiert, einer Alginatlösung (21), die auf das Gewicht bezogen einen gewissen Prozentsatz an Natriumalginat oder Kaliumalginat enthält und einer Salzwasserlösung (22), die auf das Gewicht bezogen einen gewissen Prozentsatz an divalenten Metallionen enthält;
(b) Verwenden zumindest zweier separater und sukzessiver Prozesse, um eine Schicht der Alginatlösung (21) und eine Schicht der Salzwasserlösung (22) jeweils an zumindest einer Oberfläche (11) des Filmträgers (10) abzuscheiden, wobei der erste Beschichtungsprozess durch Eintauchen durchgeführt wird und wobei der zweite Beschichtungsprozess durch Sprühbeschichtung durchgeführt wird,
(c) Querverbinden des Alginats in der Alginatlösung (21) mit divalenten Metallionen in der Salzwasserlösung (22) auf der Oberfläche (11) des Filmträgers (10), um eine Alginatmembran (20) zu erzeugen, die eine Hydrogelmembran ist, die eine Netzwerkstruktur aufweist, die an der Oberfläche (11) des Filmträgers (10) ausgebildet ist oder infiltrierend in die Oberfläche (11) des Filmträgers (10) ausgebildet ist;
wobei in Schritt (b) zuerst zumindest eine Oberfläche (11) des Filmträgers (10) mit einer Schicht der Alginatlösung (21) beschichtet wird und dann eine Schicht der Salzwasserlösung (22) über die Schicht der Alginatlösung (21) abgeschieden wird, wobei die divalenten Metallionen in der Salzwasserlösung (22) in eine innere Oberfläche des Filmträgers (10) infiltrieren werden, um das Alginat in einer Infiltrationsschnittstelle (12) quer zu verbinden, wobei eine Hydrogelmembran (20) mit einer Netzwerkstruktur über oder in der Oberfläche (11) des Filmträgers (10) ausgebildet wird, so dass die Alginatmembran (20) in die Infiltrationsschnittstelle (12) infiltriert wird und nicht leicht von dem Filmträger (10) zu trennen ist, wobei die Position der Infiltrationsschnittstelle (12) von der relativen Konzentration der Alginatlösung (21) und der Salzwasserlösung (22) abhängt;
oder wobei in dem Schritt (b) zumindest eine Oberfläche (11) des Filmträgers (10) zuerst mit einer Schicht der Salzwasserlösung (22) beschichtet wird und dann eine Schicht der Alginatlösung (21) über der Schicht der Salzwasserlösung (22) abgeschieden wird, wobei das Alginat in der Alginatlösung (21) zuerst an der Oberfläche (11) des Filmträgers (10) querverbunden wird, um eine Hydrogelmembran (20) mit höherer Dichte auszubilden, so dass die Alginatmembran (20) mit der Oberfläche (11) überlappt ist und nicht in die innere Oberfläche des Filmträgers (10) infiltriert, so dass die Hydrogelkomplexalginatmembran (20), die eine Netzwerkstruktur aufweist nur an der oberen Oberfläche (11) des Filmträgers (10) ausgebildet wird, so dass die Alginatmembran (20) von dem Filmträger (10) trennbar ist.

12. Verfahren nach Anspruch 11, bei dem der Filmträger (10) ein Vlies, Textil, ein poröser Schwamm oder ein Plastikfilm ist.

13. Verfahren nach Anspruch 11, bei dem der Filmträger (10) aus synthetischen Fasern, natürlichen Fasern, hochmolekularen Polymeren oder ihren Kombinationen hergestellt ist.

14. Verfahren nach Anspruch 11, bei dem die synthetischen Fasern Polyethylenterephthalate (PET), Nylon, Acryl, Polypropylen (PP) und Polymilchsäure (PLA) umfassen.

15. Verfahren nach Anspruch 11, bei dem der gewisse auf das Gewicht bezogene Prozentsatz an Natriumalginat oder Kaliumalginat in der Alginatlösung (21) im Bereich von 0,01% bis 20% liegt

16. Verfahren nach Anspruch 11, bei dem der gewisse auf das Gewicht bezogene Prozentsatz an Natriumalginat oder Kaliumalginat in der Alginatlösung (21) im Bereich von 1% bis 5% liegt.

17. Verfahren nach Anspruch 11, bei dem die Salzwasserlösung (22), die auf das Gewicht bezogen einen gewissen Prozentsatz der divalenten Metallionen enthält, Kalziumlactatlösung, Kalziumchloridlösung, Kalziumgluconatlösung, Polyglutaminsäurekalziumlösung, Kalziumkarbonatlösung und Kalziumsulfatlösung enthält.

18. Verfahren nach Anspruch 11, bei dem der gewisse auf das Gewicht bezogene Prozentsatz der divalenten Metallionen im Bereich von 0,01% bis 50% liegt.

19. Verfahren nach Anspruch 11, bei dem der gewisse auf das Gewicht bezogene Prozentsatz der divalenten Metallionen im Bereich von 1% bis 10% liegt.

20. Verfahren nach Anspruch 11, bei dem in dem Schritt (b) das zweite Beschichten durch Sprühbeschichtung durchgeführt wird.

21. Verfahren nach Anspruch 11, bei dem, nachdem das Querverbinden im Schritt (c) abgeschlossen ist, das Verfahren ferner einen Schritt der Durchführung eines Sprühbeschichtungsprozesses der Alginatlösung (21) umfasst und einen Sprühbeschichtungsprozess der Salzwasserlösung (22) in Reihenfolge an der Oberfläche (11) des Filmträgers (10), um quer zu verbinden und die Dicke der Alginatmembran (20) zu erhöhen.

22. Verfahren nach Anspruch 11, bei dem die Alginatlösung (21) mit Additiven zugesetzt wird, die aus der Gruppe ausgewählt sind, die aus Nährstoffen zum Gebrauch in der Kosmetik, Essenzen zum Gebrauch in der Kosmetik, und Wirkstofflösungen zum Gebrauch in der Kosmetik oder Pigmenten besteht.

## Revendications

1. Film (1) comprenant un support de film (10) et au moins une membrane d'alginate (20); dans lequel le support de film (10) est un support qui absorbe du liquide;
la membrane d'alginate (20) est une membrane hydrogel ayant une structure réticulaire formée par réticulation se présentant entre l'alginate dans au moins une couche de solution d'alginate (21) et les ions métalliques divalents d'au moins une couche de solution d'eau salée (22) appliquée sur au moins une surface (11) du support de film (10); la solution d'alginate (21) contenant un certain pourcentage d'alginate de sodium ou d'alginate de potassium en poids tandis que la solution d'eau salée (22) contenant un certain pourcentage d'ions métalliques divalents en poids; la couche de la solution d'alginate (21) et la couche de la solution d'eau salée (22) étant appliquées sur la surface (11) du support de film (10) par deux procédés de revêtement séparés et continus, dans lequel la membrane d'alginate (20) est formée sur et / ou pénétrant dans la surface du support de film (10),
dans lequel la membrane d'alginate (20) n'est pas facilement séparable du support de film (10) après tout d'abord revêtement d'au moins une surface (11) du support de film (10) avec une couche de la solution d'alginate (21) par trempage et ensuite revêtement avec une couche de la solution d'eau salée (22) sur la couche de la solution d'alginate (21) par revêtement par pulvérisation, dans lequel les ions métalliques divalents dans la solution d'eau salée (22) étant infiltrés dans une surface interne du support de film (10), de sorte que la membrane hydrogel d'alginate (20) ayant une structure réticulaire est formée au-dessus de la surface (11) du support de film (10) et dans une interface d'infiltration (12) pénétrant dans une région de surface du support de film (10), de telle manière que la membrane d'alginate (20) étant infiltrée dans le support de film (10), dans lequel la position de l'interface d'infiltration (12) est dépendante de la concentration relative de la solution d'alginate (21) et de la solution d'eau salée (22);
ou dans lequel la membrane hydrogel (20) est séparable du support de film (10) après tout d'abord revêtement d'au moins une surface (11) du support de film (10) avec une couche de la solution d'eau salée (22) par trempage et un second revêtement avec une couche d'une solution d'alginate (21) sur la couche de la solution d'eau salée (22) par revêtement par pulvérisation, dans lequel l'alginate dans la solution d'alginate (21) étant réticulé avec les ions métalliques divalents dans la solution d'eau salée (22) sur la surface (11) du support de film (10) pour former une membrane hydrogel (20) ayant une masse volumique supérieure, dans lequel l'interface d'infiltration (12) étant chevauchée par la surface (11) et ne s'infiltrant pas dans la surface interne du support de film (10), dans lequel la membrane hydrogel (20) ayant une structure réticulaire seulement formée sur la surface supérieure (11) du support de film (10).

2. Film selon la revendication 1, dans lequel le support de film (10) est un tissu non tissé, un textile, un film d'éponge poreux ou un film plastique.

3. Film selon la revendication 1, dans lequel le support de film (10) est fait de fibres synthétiques, de fibres naturelles, de polymères à poids moléculaire élevé ou de leurs combinaisons.

4. Film selon la revendication 3, dans lequel les fibres synthétiques incluent le téréphtalate de polyéthylène (PET), le nylon, les composés acryliques, le polypropylène (PP) et le poly(acide lactique) (PLA).

5. Film selon la revendication 1, dans lequel le certain pourcentage d'alginate de sodium ou d'alginate de potassium dans la solution d'alginate (21) se situe dans la plage de 0,01 % à 20 % en poids.

6. Film selon la revendication 1, dans lequel le certain pourcentage d'alginate de sodium ou d'alginate de potassium dans la solution d'alginate (21) se situe dans la plage de 1 % à 5 % en poids.

7. Film selon la revendication 1, dans lequel la solution d'eau salée (22) contenant un certain pourcentage des ions métalliques divalents en poids inclut une solution de lactate de calcium, une solution de chlorure de calcium, une solution de gluconate de calcium, une solution de poly(acide glutamique) calcique, une solution de carbonate de calcium et une solution de sulfate de calcium.

8. Film selon la revendication 1, dans lequel le certain pourcentage des ions métalliques divalents se situe dans la plage de 0,01 % à 50 % en poids.

9. Film selon la revendication 1, dans lequel le certain pourcentage des ions métalliques divalents se situe dans la plage de 1 % à 10 % en poids.

10. Film selon la revendication 1, dans lequel la solution d'alginate (21) est complétée avec des additifs choisis dans le groupe constitué de incluant les nutriments à usage cosmétique, les essences à usage cosmétique et les solutions pharmaceutiques à usage cosmétique ou les pigments.

11. Procédé de fabrication d'un film (1) comprenant un support de film (10) et au moins une membrane d'alginate (20) comprenant les étapes de:
(a) fourniture d'un support de film (10) qui absorbe du liquide, d'une solution d'alginate (21) contenant un certain pourcentage d'alginate de sodium ou d'alginate de potassium en poids et d'une solution d'eau salée (22) contenant un certain pourcentage d'ions métalliques divalents en poids;
(b) utilisation d'au moins deux procédés séparés et successifs pour appliquer une couche de la solution d'alginate (21) et une couche de la solution d'eau salée (22) respectivement sur au moins une surface (11) du support de film (10), dans lequel le premier procédé de revêtement est réalisé par trempage; et dans lequel le second procédé de revêtement est réalisé par revêtement par pulvérisation;
(c) réticulation de l'alginate dans la solution d'alginate (21) avec les ions métalliques divalents dans la solution d'eau salée (22) sur la surface (11) du support de film (10) de façon à produire une membrane d'alginate (20) qui est une membrane hydrogel ayant une structure réticulaire, formée sur la surface (11) du support de film (10), ou formée en s'infiltrant sur la surface (11) du support de film (10);
dans lequel dans l'étape (b), tout d'abord au moins une surface (11) du support de film (10) est revêtue avec une couche de la solution d'alginate (21) et ensuite une couche de la solution d'eau salée (22) est appliquée sur la couche de la solution d'alginate (21), dans lequel les ions métalliques divalents dans la solution d'eau salée (22) s'infiltreront dans une surface interne du support de film (10) en vue de la réticulation de l'alginate à l'intérieur d'une interface d'infiltration (12), dans lequel une membrane hydrogel (20) ayant une structure réticulaire est formée au-dessus de ou dans la surface (11) du support de film (10), de sorte que la membrane d'alginate (20) est infiltrée dans l'interface d'infiltration (12) et n'étant pas facilement séparable du support de film (10), dans lequel la position de l'interface d'infiltration (12) dépend de la concentration relative de la solution d'alginate (21) et de la solution d'eau salée (22);
ou dans lequel dans l'étape (b), au moins une surface (11) du support de film (10) est tout d'abord revêtue avec une couche de la solution d'eau salée (22) et ensuite une couche d'une solution d'alginate (21) est appliquée sur la couche de la solution d'eau salée (22), dans lequel l'alginate dans la solution d'alginate (21) est tout d'abord réticulé au niveau de la surface (11) du support de film (10) pour former une membrane hydrogel (20) ayant une masse volumique supérieure, de sorte que la membrane d'alginate (20) est chevauchée par la surface (11) et ne s'infiltre pas dans la surface interne du support de film (10), de sorte que la membrane hydrogel complexe d'alginate (20) ayant une structure réticulaire est seulement formée sur la surface supérieure (11) du support de film (10), de sorte que la membrane d'alginate (20) est séparable du support de film (10).

12. Procédé selon la revendication 11, dans lequel le support de film (10) est un tissu non tissé, un textile, un film d'éponge poreux ou un film plastique.

13. Procédé selon la revendication 11, dans lequel le support de film (10) est fait de fibres synthétiques, de fibres naturelles, de polymères à poids moléculaire élevé ou de leurs combinaisons.

14. Procédé selon la revendication 11, dans lequel les fibres synthétiques incluent le téréphtalate de polyéthylène (PET), le nylon, les composés acryliques, le polypropylène (PP) et le poly(acide lactique) (PLA).

15. Procédé selon la revendication 11, dans lequel le certain pourcentage d'alginate de sodium ou d'alginate de potassium dans la solution d'alginate (21) se situe dans la plage de 0,01 % à 20 % en poids.

16. Procédé selon la revendication 11, dans lequel le certain pourcentage d'alginate de sodium ou d'alginate de potassium dans la solution d'alginate (21) se situe dans la plage de 1 % à 5 % en poids.

17. Procédé selon la revendication 11, dans lequel la solution d'eau salée (22) contenant un certain pourcentage des ions métalliques divalents en poids inclut une solution de lactate de calcium, une solution de chlorure de calcium, une solution de gluconate de calcium, une solution de poly(acide glutamique) calcique, une solution de carbonate de calcium et une solution de sulfate de calcium.

18. Procédé selon la revendication 11, dans lequel le certain pourcentage des ions métalliques divalents se situe dans la plage de 0,01 % à 50 % en poids.

19. Procédé selon la revendication 11, dans lequel le certain pourcentage des ions métalliques divalents se situe dans la plage de 1 % à 10 % en poids.

20. Procédé selon la revendication 11, dans lequel dans l'étape (b), le second revêtement est réalisé par revêtement par pulvérisation.

21. Procédé selon la revendication 11, dans lequel après que la réticulation dans l'étape (c) est terminée, le procédé inclut en outre une étape de réalisation d'un procédé de revêtement par pulvérisation de la solution d'alginate (21) et un procédé de revêtement par pulvérisation de la solution d'eau salée (22) dans l'ordre sur la surface (11) du support de film (10) en vue de la réticulation et de l'augmentation de l'épaisseur de la membrane d'alginate (20).

22. Procédé selon la revendication 11, dans lequel une solution d'alginate (21) est complétée avec des additifs choisis dans le groupe constitué des nutriments à usage cosmétique, des essences à usage cosmétique et des solutions pharmaceutiques à usage cosmétique ou des pigments.
